# EUROPEAN PATENT APPLICATION

(11) **EP 3 771 402 A1**
(43) Date of publication of application: **03.02.2021**
(21) Application number: 19189277.7
(22) Date of filing: 31.07.2019
(51) Int. Cl.: A61B 5/022, A61B 5/021, A61B 5/00

(54) **APPARATUS FOR USE WITH A WEARABLE CUFF IN MEASURING BLOOD PRESSURE**

(71) Applicant: Koninklijke Philips N.V., 5656 AG Eindhoven (NL)
(72) Inventor: DERKX, Rene Martinus Maria, 5656 AE Eindhoven (NL); LAMICHHANE, Bishal, 5656 AE Eindhoven (NL); KUENEN, Maarten Petrus Joseph, 5656 AE Eindhoven (NL)
(74) Representative: de Haan, Poul Erik

(57) **Abstract**

There is provided an apparatus (12). The apparatus (12) comprises one or more processors configured to receive a pressure signal indicative of a cuff (14) pressure during cuff inflation and subsequent cuff deflation, and a first oscillation signal indicative of cuff pressure oscillations at different cuff pressures during cuff inflation. The one or more processors are configured to detect whether the first oscillation signal comprises at least one artifact and, if so, identify one or more cuff pressures at which the at least one artifact is detected, control a valve to release pressure from the cuff (14) during cuff deflation and receive a second oscillation signal indicative of cuff pressure oscillations at the one or more identified cuff pressures during cuff deflation. The one or more processors are configured to process the pressure signal, the first oscillation signal and the second oscillation signal to measure a blood pressure.

## Description

### FIELD OF THE INVENTION

The disclosure relates to an apparatus for use with a wearable cuff in measuring blood pressure and a method of operating the apparatus.

### BACKGROUND OF THE INVENTION

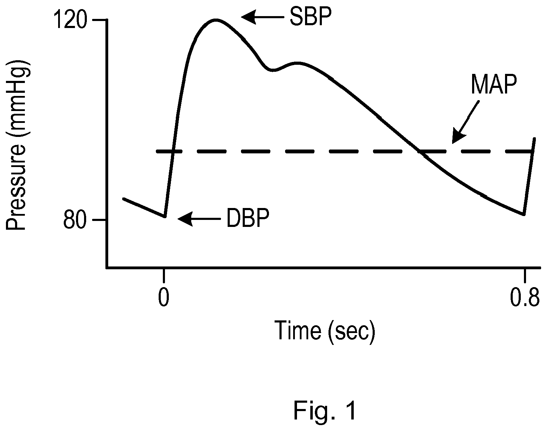

Blood pressure (BP) or, more precisely, arterial blood pressure, is the pressure exerted by circulating blood on the arterial vessel walls. Blood pressure in arteries varies with the pulsation of the blood by the heart. It is one of the key vital signs to establish patient well-being and thus needs to be monitored. Blood pressure is a periodic signal, which rises at each contraction of the heart and decreases in between heart beats. It is typically described by systolic blood pressure (SBP), diastolic blood pressure (DBP) and mean arterial blood pressure (MAP), where systolic blood pressure is the maximum blood pressure during a heart cycle, diastolic blood pressure is the minimum blood pressure during a heart cycle, and mean arterial blood pressure is the average blood pressure during a heart cycle or, more specifically, the time averaged blood pressure during a heart cycle. Fig. 1 illustrates a blood pressure waveform from which DBP, SBP and MAP measurements can be obtained.

Different techniques exist by which blood pressure can be determined and these can be classified as invasive or non-invasive blood pressure measurement techniques. An example of an invasive blood pressure measurement technique is where, during a single heart-cycle, blood pressure is measured in a major artery (e.g. the brachial artery in the arm) invasively using an arterial line. Typically, non-invasive blood pressure (NIBP) measurement techniques are cuff-based, which require an inflatable (e.g. pneumatic) cuff to be placed around a limb (which is usually the upper arm) of a subject. The pressure in the cuff is then changed to infer blood pressure. A common method that uses a cuff in this way is referred to in the art as the oscillometric method.

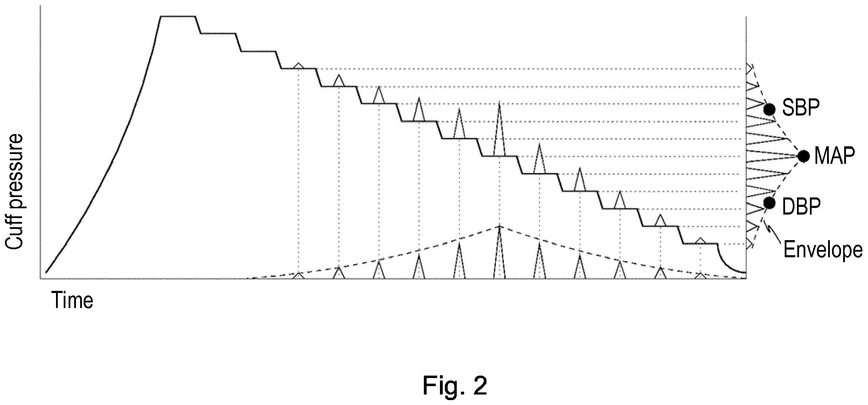

In the oscillometric method, a cuff is inflated around a limb of a subject until the pressure in the cuff is larger than the SBP and the artery is fully occluded. After this has happened, the pressure in the cuff is decreased in a step-wise manner (e.g. by 3 mmHg per step), as shown in Fig. 2. The pressure in the cuff is decreased through a valve until the pressure drops below the DBP. SBP and DBP measurements are based on pressure oscillations (or alternating current, AC, pressure variations) that are induced in the cuff by each heart beat. Thus, the (AC) pressure oscillations in the cuff are evaluated as function of the cuff (direct current, DC) pressure during the stepped deflations. During the stepped deflation, the pressure oscillations during each heart-cycle can be sensed by the cuff by, for example, applying high-pass filtering, which removes the external applied pressure in the cuff. The strength of the pressure oscillations (which, for example, can be computed via min-max computation of the high-pass filtered pressure signal) vary with the applied pressure, as is shown in Fig. 2.

The amplitude of the pressure oscillations depends on the difference between the cuff pressure and the actual arterial blood pressure. The maximum amplitude of the pressure oscillations is present at the point where the externally applied cuff pressure is roughly equal to the (time-averaged) internal blood pressure in the artery. In this situation, the forces on the arterial wall (averaged over time) are roughly equal on the inside and the outside and the artery is 'unloaded', which provides the largest 'swing' of the (maximally compliant) arterial-wall wall. Since the externally applied cuff pressure equals the time-averaged internal blood pressure in this case, MAP can be defined. In particular, the cuff pressure corresponding to the maximum oscillation amplitude is typically taken as the MAP. The points for SBP and DBP are less easy to define. SBP and DBP are usually determined as the cuff pressure at which a fixed ratio of the maximum oscillation amplitude occurs. These fractions are often heuristically determined. Typically, these fractions are chosen such that the DBP and SBP that is computed from the pressure oscillation envelope complies with the equation: MAP = 2/3 ^{∗} DBP + 1/3 ^{∗} SBP. Thus, the pressure oscillation envelope is computed as a function of applied pressure, as illustrated in Fig. 2, and the pressure oscillation envelope is then used in the assessment of the MAP, SBP, and DBP. The pressure oscillation envelope can, for example, be computed by fitting a curve to the pressure oscillations.

Although measuring blood pressure via stepped deflation is accurate, the cuff is generally inflated to a maximum pressure prior to the stepped deflation. This maximum pressure can either be defined a-priori, but is usually defined by determining when there are no oscillations visible anymore during the cuff inflation. The actual blood pressure measurement via stepped deflation is slow, which causes prolonged discomfort for the subject. In order to speed up the blood pressure measurement, the stepped deflation can be replaced by a continuous ramped inflation. Hence, several measurement devices use an inflation-based oscillometric measurement, where the inflation ramp is continuous (i.e. does not have steps or plateaus). In these devices, the speed of the pump used to inflate the cuff is controlled during the inflation, based on e.g. the heart-rate such that a sufficient amount of heart-beats are present in the inflation ramp to compute (accurately enough) the oscillation envelope, in order to compute MAP, DBP and SBP. The pump stops when there are no longer enough oscillations measurable and an exhaust valve is then opened to deflate the cuff for a next measurement.

However, while an inflation-based oscillometric measurement reduces the blood pressure measurement time, it is difficult to check the consistency of oscillations, since the cuff pressure is continuously changing. Moreover, due to the short measurement duration in the inflation-based oscillometric blood pressure measurement, the amount of oscillations is small and this can be an issue since it is important to obtain as many oscillations as possible in the curve-fit to obtain the oscillation envelope to be used in the assessment for the MAP, SBP and DBP. The blood pressure measurements can also comprise artifacts, such as those due to motion (e.g. when the subject moves) and/or temporal arrhythmias (e.g. a premature heart beat). There are several types of artifacts that can lead to a smaller amount of oscillations in the assessment. If the amount of oscillations is too small during the inflation of the cuff, a trivial solution is to apply (before full deflation by opening the exhaust valve) a stepped deflation as described earlier. However, this concatenation of the two measurement times (i.e. the time for the inflation-based measurement plus the time for the stepped deflation-based measurement) still results in prolonged discomfort for the subject.

A paper entitled "A Blood Pressure Monitor with Robust Noise Reduction System under Linear Cuff Inflation and Deflation", by Takashi et al., 32nd Annual International Conference of the IEEE EMBS Buenos Aires, Argentina, 2010 discloses another technique that combines two measurement modes: a linear inflation mode and a linear deflation mode. However, in this technique, once the inflation mode fails to calculate appropriate blood pressure due to body movement or arrhythmia, the blood pressure monitor switches automatically to the deflation mode. Also, when the blood pressure monitor identifies a motion artifact, it stops the cuff deflation and the oscillation candidates are excluded as noise caused by the motion artifact. Thus, while the speed of the blood pressure measurement can be improved according to this technique, the accuracy and reliability of the blood pressure measurement is reduced because some of the measurement data is lost.

### SUMMARY OF THE INVENTION

As noted above, a limitation with existing techniques for measuring blood pressure is that the accuracy and reliability of blood pressure measurements is compromised for an increased speed of blood pressure measurement, or vice versa. It would therefore be valuable to have an improvement aimed at addressing these limitations.

Therefore, according to a first aspect, there is provided an apparatus for use with a wearable cuff in measuring blood pressure. The cuff is inflatable to pressurize a measurement site of a subject. The apparatus comprises one or more processors. The one or more processors are configured to receive a pressure signal indicative of a pressure in the cuff during a period in which the cuff is inflated and subsequently deflated. The one or more processors are configured to receive a first oscillation signal indicative of pressure oscillations in the cuff at different pressures in the cuff during the period in which the cuff is inflated and detect whether the first oscillation signal comprises at least one artifact. The one or more processors are configured to, if the first oscillation signal is detected to comprise at least one artifact, identify, in the pressure signal, one or more pressures in the cuff at which the at least one artifact is detected, control a valve to release pressure from the cuff during the period in which the cuff is subsequently deflated and receive a second oscillation signal indicative of pressure oscillations in the cuff at the one or more identified pressures during the period in which the cuff is subsequently deflated. The one or more processors are configured to process the pressure signal, the first oscillation signal and the second oscillation signal to measure a blood pressure of a subject.

In some embodiments, the one or more processors may be configured to control the valve to maintain a constant or substantially constant pressure in the cuff during the period in which the cuff is subsequently deflated when the pressure in the cuff reaches the one or more identified pressures. In some embodiments, the one or more processors may be configured to control the valve to release pressure from the cuff continuously during the period in which the cuff is subsequently deflated apart from one or more time periods in which the constant or substantially constant pressure is maintained in the cuff. In some embodiments, the one or more processors may be configured to control the valve to maintain the constant or substantially constant pressure in the cuff for a time period that covers at least one pressure oscillation that is free from artifacts and/or that covers at least one heart cycle.

In some embodiments, the one or more processors may be configured to process the pressure signal and the first and second oscillation signals to measure the blood pressure of the subject by being configured to measure, from the first oscillation signal, a plurality of first pressure oscillation amplitudes at the different pressures in the cuff, measure, from the second oscillation signal, one or more second pressure oscillation amplitudes at the one or more identified pressures in the cuff, combine the one or more second pressure oscillation amplitudes with the plurality of first pressure oscillation amplitudes to obtain a set of combined pressure oscillation amplitudes at the different pressures in the cuff, and process the set of combined pressure oscillation amplitudes at the different pressures in the cuff to measure the blood pressure of the subject.

In some embodiments, the one or more processors may be configured to combine the one or more second pressure oscillation amplitudes with the plurality of first pressure oscillation amplitudes by being configured to replace one or more of the plurality of first pressure oscillation amplitudes at the one or more identified pressures in the cuff with the one or more corresponding second pressure oscillation amplitudes to obtain the set of combined pressure oscillation amplitudes. In some embodiments, the one or more processors may be configured to replace the one or more of the plurality of first pressure oscillation amplitudes by being configured to compare the one or more of the plurality of first pressure oscillation amplitudes to the one or more corresponding second pressure oscillation amplitudes and, for each discrepancy identified from the comparison, replace the one or more of the plurality of first pressure oscillation amplitudes with the one or more corresponding second pressure oscillation amplitudes.

In some embodiments, the one or more processors may be configured to acquire, from at least one sensor, data indicative of a cause of the at least one artifact during the period in which the cuff is inflated and detect whether the first oscillation signal comprises at least one artifact based on the acquired data. In some embodiments, the one or more processors may be configured to detect whether the first oscillation signal comprises at least one artifact based on the acquired data by being configured to compare the acquired data to the pressure signal and/or the first oscillation signal to detect whether the first oscillation signal comprises at least one artifact. In some embodiments, the at least one sensor may comprise a motion sensor and the acquired data may comprise motion data indicative of a motion of the cuff during the period in which the cuff is inflated and/or the at least one sensor may comprise an electrocardiogram sensor and the acquired data may comprise electrocardiogram data indicative of an irregular heart beat of the subject during the period in which the cuff is inflated.

In some embodiments, the one or more processors may be configured to control the inflation of the cuff at a predetermined speed and/or up to a maximum pressure prior to subsequent deflation.

In some embodiments, the measured blood pressure of the subject may comprise a mean arterial pressure of the subject, a systolic blood pressure of the subject and/or a diastolic blood pressure of the subject.

According to a second aspect, there is provided a system comprising the apparatus as described earlier. In some embodiments, the system may also comprises any one or more of the cuff and the valve.

According to a third aspect, there is provided a method of operating an apparatus for use with a wearable cuff in measuring blood pressure. The cuff is inflatable to pressurize a measurement site of a subject. The method comprises receiving a pressure signal indicative of a pressure in the cuff during a period in which the cuff is inflated and subsequently deflated. The method comprises receiving a first oscillation signal indicative of pressure oscillations in the cuff at different pressures in the cuff during the period in which the cuff is inflated and detecting whether the first oscillation signal comprises at least one artifact. The method comprises, if the first oscillation signal is detected to comprise at least one artifact, identifying, in the pressure signal, one or more pressures in the cuff at which the at least one artifact is detected, controlling a valve to release pressure from the cuff during the period in which the cuff is subsequently deflated, and receiving a second oscillation signal indicative of pressure oscillations in the cuff at the one or more identified pressures during the period in which the cuff is subsequently deflated. The method comprises processing the pressure signal, the first oscillation signal and the second oscillation signal to measure a blood pressure of the subject.

According to a fourth aspect, there is provided a computer program product comprising a computer readable medium. The computer readable medium has a computer readable code embodied therein. The computer readable code is configured such that, on execution by a suitable computer or processor, the computer or processor is caused to operate the apparatus according to the method described earlier.

According to the aspects and embodiments described above, the limitations of existing techniques are addressed. In particular, it has been appreciated that any pressure oscillation at which an artifact is detected at one or more identified pressures during inflation of the cuff may be inaccurate and leads to a smaller amount of pressure oscillations which decreases the accuracy of the blood pressure measurement. Thus, according to the above-described aspects and embodiments, this is accounted for by the acquisition of a second oscillation signal indicative of pressure oscillations in the cuff at the same one or more identified pressures during deflation of the cuff. This means that the second oscillation signal will comprise reliable pressure oscillations in the deflation phase that can be used to check and/or even replace the corresponding potentially unreliable pressure oscillations of the first oscillation signal in the inflation phase where the artifact occurred.

The first and second oscillation signals according to the above-described aspects and embodiments thus provide an accurate and more reliable indication of the pressure oscillations at the different pressures in the cuff and, as such, the blood pressure measured using the pressure signal and these first and second oscillation signals is also more accurate and reliable. At the same time, this improved accuracy and reliability is achieved without compromising on the speed of a blood pressure measurement, since the inflation and deflation of the cuff is as efficient as possible with re-measurement of pressure oscillations during deflation only being needed at the one or more identified pressures at which an artifact is detected during the inflation. There is thus provided an improved technique for use in measuring blood pressure.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a graphical illustration of an arterial waveform;
Fig. 2 is a graphical illustration of pressure oscillations obtained during stepped deflation;
Fig. 3 is a schematic illustration of an apparatus according to an embodiment;
Figs. 4 and 5 are schematic illustrations of systems according to embodiments;
Fig. 6 is a flow chart illustrating a method according to an embodiment;
Fig. 7 is a graphical illustration of an example pressure signal;
Fig. 8 is a graphical illustration of pressure oscillations obtained during inflation and deflation; and
Figs. 9, 10 and 11 are graphical illustrations of pressure oscillation signals and corresponding pressure envelopes obtained during inflation.

### DETAILED DESCRIPTION OF EMBODIMENTS

As noted above, there is provided herein an improved technique for measuring blood pressure. In more detail, there is provided an apparatus for use with a wearable cuff (or clamp unit) in measuring blood pressure, which overcomes the limitation associated with existing techniques. The apparatus described herein is for use in measuring blood pressure non-invasively. That is, the apparatus described herein is a non-invasive blood pressure (NIBP) apparatus (or a NIBP measurement apparatus). In some embodiments, the apparatus described herein can be a server (e.g. a decision server), or any other type of apparatus.

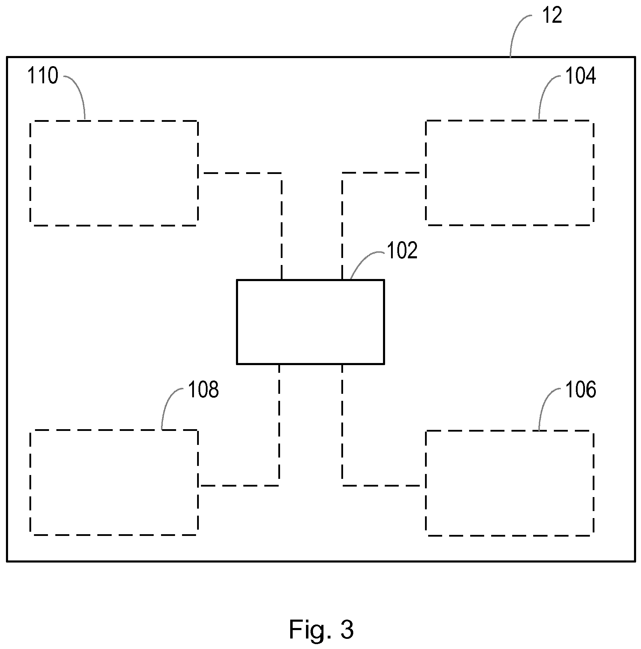

Fig. 3 illustrates the apparatus 12 for use with a wearable cuff in measuring blood pressure according to an embodiment. As illustrated in Fig. 3, the apparatus 12 comprises one or more processors 102. The one or more processors 102 can be implemented in numerous ways, with software and/or hardware, to perform the various functions described herein. In particular implementations, the one or more processors 102 can comprise a plurality of software and/or hardware modules, each configured to perform, or that are for performing, individual or multiple steps of the method described herein. The one or more processors 102 may comprise, for example, one or more microprocessors, one or more multicore processors, one or more digital signal processors (DSPs), one or more processing units, and/or one or more controllers (e.g. one or more microcontrollers) that may be configured or programmed (e.g. using software or computer program code) to perform the various functions described herein. The one or more processors 102 may be implemented as a combination of dedicated hardware (e.g. amplifiers, pre-amplifiers, analog-to-digital convertors (ADCs) and/or digital-to-analog convertors (DACs)) to perform some functions and one or more processors (e.g. one or more programmed microprocessors, DSPs and associated circuitry) to perform other functions.

Briefly, the one or more processors 102 of the apparatus 12 are configured to receive a pressure signal indicative of a pressure in the cuff during a period in which the cuff is inflated and subsequently deflated. The one or more processors 102 of the apparatus 12 are configured to receive a first oscillation signal indicative of pressure oscillations in the cuff at (a plurality of) different pressures in the cuff during the period in which the cuff is inflated and detect whether the first oscillation signal comprises at least one artifact. The one or more processors 102 of the apparatus 12 are configured to, if the first oscillation signal is detected to comprise at least one artifact, identify in the pressure signal one or more pressures in the cuff at which the at least one artifact is detected, control a valve to release pressure from the cuff during a period in which the cuff is deflated and receive a second oscillation signal indicative of pressure oscillations in the cuff at the one or more identified pressures during the period in which the cuff is deflated. The one or more processors 102 of the apparatus 12 are configured to process the pressure signal, the first oscillation signal and the second oscillation signal to measure a blood pressure of a subject.

In some embodiments, the one or more processors 102 of the apparatus 12 may comprise any one or more of an inflation controller configured to control the inflation of the cuff, an artifact detection module to detect whether the first oscillation signal comprises at least one artifact, a deflation controller configured to control the deflation of the cuff (or, more specifically, the valve to release pressure from the cuff to control the deflation of the cuff), a blood pressure measurement module configured to process the pressure signal and the first and second oscillation signals to measure the blood pressure of the subject, or any other processor (e.g. controller or module), or any combination of processors (e.g. any one or more controllers and/or any one or more modules).

Any references herein to "pressure signal" or "received pressure signal" will be understood to mean a pressure signal or a received pressure signal indicative of the pressure in the cuff during a period for measuring blood pressure. More specifically, the pressure signal or received pressure signal referred to herein can be indicative of the (e.g. slowly) varying direct current (DC) pressure during the period for measuring blood pressure. Similarly, any references herein to "first oscillation signal", "received first oscillation signal", "second oscillation signal" and "received second oscillation signal" will be understood to mean an oscillation signal or a received oscillation signal indicative of pressure oscillations in the cuff during a period for measuring blood pressure. The pressure oscillations in the cuff will be understood to mean oscillations in pressure in the cuff. More specifically, the pressure oscillations in the cuff can be (e.g. quick) alternating current (AC) pressure variations in the cuff. The oscillations in pressure in the cuff result from the heart beat of the subject.

Herein, the valve that is controlled to release pressure from the cuff during the period in which the cuff is deflated may also be referred to as a controllable valve, an exhaust valve, a controllable exhaust valve, a deflation valve or a controllable deflation valve. The subject referred to herein can be any subject, for example, an adult or a pediatric subject (e.g. an infant, a child or an adolescent). An infant can, for example, be a neonate (such as a pre-term or premature infant), a full-term infant or a post-term infant.

As illustrated in Fig. 3, in some embodiments, the apparatus 12 may comprise at least one pressure sensor 104. Alternatively or in addition, in some embodiments, at least one pressure sensor 104 may be external to (e.g. separate to or remote from) the apparatus 12. For example, another apparatus may comprise at least one pressure sensor 104 according to some embodiments. In some embodiments, the cuff may comprise at least one pressure sensor 104. Generally, the at least one pressure sensor 104 is configured to measure pressure and pressure oscillations. More specifically, the at least one pressure sensor 104 can be configured to obtain the pressure signal, the first oscillation signal, and the second oscillation signal. For example, the at least one pressure sensor 104 can be configured to measure the pressure in the cuff 14 and the pressure oscillations in the cuff 14. The one or more processors 102 of the apparatus 12 can be configured to receive the pressure signal, the first oscillation signal, and the second oscillation signal from the at least one pressure sensor 104.

In some embodiments, the at least one pressure sensor 104 can comprise a first pressure sensor configured to measure the pressure signal and a second pressure sensor configured to measure the first and second oscillation signals. For example, in some embodiments, the first pressure sensor can be configured to measure the (e.g. slowly) varying (DC) pressures in the cuff 14 to obtain the pressure signal and the second pressure sensor can be configured to measure the (e.g. quickly) varying (AC) pressure oscillations in the cuff 14 to obtain the first and second oscillation signals. Alternatively, the at least one pressure sensor 104 can comprise a single pressure sensor configured to measure the pressure signal and the first and second oscillation signals. For example, in some embodiments, the single pressure sensor can be configured to measure an overall pressure signal (e.g. comprising both the DC and AC pressures in the cuff 14) and derive the pressure signal by low-pass filtering the overall pressure signal (e.g. to derive the DC component of that overall signal) and the first and second oscillation signals by high-pass filtering the overall pressure signal (e.g. to derive the AC component of that overall signal). The one or more processors 102 of the apparatus 12 may communicate with and/or connect to the at least one pressure sensor 104 in any suitable way.

As illustrated in Fig. 3, in some embodiments, the apparatus 12 may comprise at least one memory 106. Alternatively or in addition, at least one memory 106 may be external to (e.g. separate to or remote from) the apparatus 12. For example, a hospital database may comprise the memory, the memory may be a cloud computing resource, or similar. The one or more processors 102 of the apparatus 12 may be configured to communicate with and/or connect to at least one memory 106. A memory may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). In some embodiments, at least one memory 106 can be configured to store program code that can be executed by the one or more processors 102 of the apparatus 12 to cause the apparatus 12 to operate in the manner described herein.

Alternatively or in addition, in some embodiments, at least one memory 106 can be configured to store information required by or resulting from the method described herein. For example, in some embodiments, at least one memory 106 may be configured to store any one or more of the pressure signal, the first oscillation signal, an indication of whether the first oscillation signal is detected to comprise at least one artifact, the one or more identified pressures at which the at least one artifact is detected, the second oscillation signal and the measured blood pressure of the subject, or any other information, or any combination of information, required by or resulting from the method described herein. In some embodiments, the one or more processors 102 of the apparatus 12 can be configured to control at least one memory 106 to store information required by or resulting from the method described herein.

As illustrated in Fig. 3, in some embodiments, the apparatus 12 may comprise a user interface 108. Alternatively or in addition, the user interface 108 may be external to (e.g. separate to or remote from) the apparatus 12. The one or more processors 102 of the apparatus 12 may be configured to communicate with and/or connect to a user interface 108. In some embodiments, the one or more processors 102 of the apparatus 12 can be configured to control the user interface 108 to operate in the manner described herein.

The user interface 108 can be configured to render (or output, display, or provide) information required by or resulting from the method described herein. For example, in some embodiments, the user interface 108 may be configured to render (or output, display, or provide) any one or more of the pressure signal, the first oscillation signal, an indication of whether the first oscillation signal is detected to comprise at least one artifact, the one or more identified pressures at which the at least one artifact is detected, the second oscillation signal and the measured blood pressure of the subject, or any other information, or any combination of information, required by or resulting from the method described herein. Alternatively or in addition, the user interface 108 can be configured to receive a user input. For example, the user interface 108 may allow a user to manually enter information or instructions, interact with and/or control the apparatus 12. Thus, the user interface 108 may be any user interface that enables the rendering (or outputting, displaying, or providing) of information and, alternatively or in addition, enables a user to provide a user input.

For example, the user interface may comprise one or more switches, one or more buttons, a keypad, a keyboard, a mouse, a touch screen or an application (for example, on a smart device such as a tablet, a smartphone, or any other smart device), a display or display screen, a graphical user interface (GUI) such as a touch screen, or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface that is controlled to render information may be the same user interface as that which enables the user to provide a user input.

As illustrated in Fig. 3, in some embodiments, the apparatus 12 may comprise a communications interface (or communications circuitry) 110. Alternatively or in addition, the communications interface 110 may be external to (e.g. separate to or remote from) the apparatus 12. The communications interface 110 can be for enabling the apparatus 12, or components (e.g. the one or more processors 102, the at least one pressure sensor 104, the at least one memory 106, the user interface 108, the valve and/or a pump) of the apparatus 12, to communicate with and/or connect to one or more other components, sensors, interfaces, devices, or memories (such as any of those described herein). For example, the communications interface can be for enabling the one or more processors of the apparatus 12 to communicate with and/or connect to the at least one pressure sensor 104, the at least one memory 106 and/or the user interface 108.

The communications interface may enable the apparatus 12, or components of the apparatus 12, to communicate and/or connect in any suitable way. For example, the communications interface may enable the apparatus 12, or components of the apparatus 12, to communicate and/or connect wirelessly, via a wired connection, or via any other communication (or data transfer) mechanism. In some wireless embodiments, for example, the communications interface may enable the apparatus 12, or components of the apparatus 12, to use radio frequency (RF), Bluetooth, or any other wireless communication technology to communicate and/or connect.

Although not illustrated in Fig. 3, in some embodiments, the apparatus 12 may comprise a pump. Alternatively or in addition, a pump may be external to (e.g. separate to or remote from) the apparatus 12. The pump can be controllable to inflate the cuff 14. A pump can thus be any pump that is controllable to inflate the cuff 14. In some embodiments, the pump can be controllable by the one or more processors 102 of the apparatus 12 to inflate the cuff 14. The one or more processors 102 of the apparatus 12 may communicate with and/or connect to the pump in any suitable way to control the pump.

Although also not illustrated in Fig. 3, in some embodiments, the apparatus 12 may comprise the valve. Alternatively, the valve may be external to (e.g. separate to or remote from) the apparatus 12. In some embodiments, cuff 14 may comprise the valve. In other embodiments, a tube (or hose) connected to the cuff 14 may comprise the valve. The valve is controllable to deflate the cuff 14. The valve can thus be any valve that is controllable to deflate the cuff 14. The one or more processors 102 of the apparatus 12 may communicate with and/or connect to the valve in any suitable way to control the valve.

Although also not illustrated in Fig. 3, in some embodiments, the apparatus 12 may comprise the at least one other sensor. Alternatively, the at least one other sensor may be external to (e.g. separate to or remote from) the apparatus 12. The at least one other sensor can be configured to obtain data indicative of a cause of the at least one artifact during the period in which the cuff 14 is inflated. The at least one other sensor can be a sensor besides (or in addition to) the at least one pressure sensor 104. Thus, according to some embodiments, the apparatus 12 can comprise at least one first sensor and at least one second sensor, where the at least one first sensor comprises the at least one pressure sensor 104 and the at least one second sensor comprises the at least one other sensor.

The at least one other sensor can, for example, comprise one or more motion sensors (e.g. one or more accelerometers), one or more electrocardiogram (ECG) sensors, or any other sensor, or combination of sensors, suitable for obtaining data indicative of a cause of the at least one artifact during the period in which the cuff 14 is inflated. In embodiments involving a motion sensor, the obtained data can comprise motion data indicative of a motion of the cuff 14 during the period in which the cuff 14 is inflated. Thus, the one or more motion sensors can be configured to detect motion artifacts (e.g. due to subject movement). In embodiments involving an ECG sensor, the obtained data can comprise ECG data indicative of an irregular heart beat of the subject 18 during the period in which the cuff 14 is inflated. Thus, the one or more ECG sensors can be configured to detect irregular heartbeats (such as arrhythmia, e.g. premature heart beats) as artifacts.

Other artifacts may also be detected in a similar manner. For example, artifacts due to changes in the speed at which the cuff is inflated (e.g. a speed setting of the pump 24) can be detected and/or artifacts due to cuff slippage (e.g. the loosening of Velcro used to fasten the cuff 14) can be detected, such as by detecting a sudden decrease in pressure in the cuff 14. The at least one other sensor 30 can be configured to provide the obtained data to the one or more processors 102 of the apparatus 12 (e.g. the artifact detection module 32). In this way, the one or more processors 102 of the apparatus 12 (e.g. the artifact detection module 32) can acquire the data from the least one other sensor 30.

Although also not illustrated in Fig 3, the apparatus 12 may comprise a battery or other power supply for powering the apparatus 12 or means for connecting the apparatus 12 to a mains power supply. It will also be understood that the apparatus 12 may comprise any other component to those described herein or any combination of components.

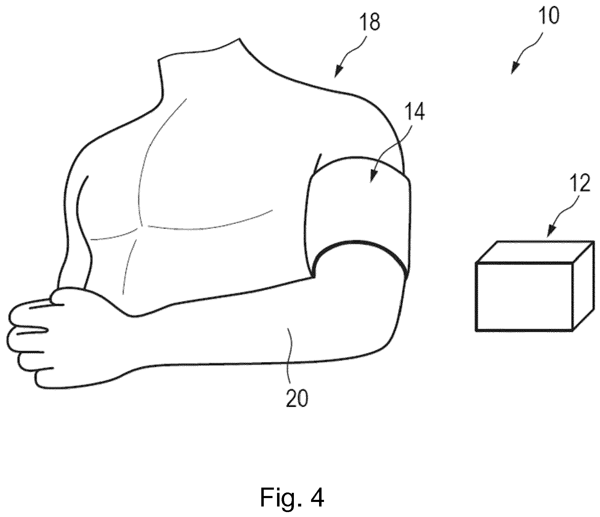

Fig. 4 illustrates the apparatus 12 in use with a wearable cuff 14 according to an embodiment. There is thus provided a system 10 comprising the apparatus 12 and the cuff 14. The cuff 14 referred to herein is for use in measuring blood pressure. The cuff 14 described herein can be for use in measuring blood pressure non-invasively. That is, the cuff 14 described herein can be a NIBP cuff (or a NIBP measurement cuff) according to some embodiments. The cuff 14 is inflatable to pressurize a measurement site 20 of a subject (e.g. a patient). In this way, the cuff 14 can pressurize an artery in the measurement site 20 of the subject 18. Although not illustrated, the cuff 14 referred to herein can also be inflatable to pressurize a phantom (or dummy) of a body part, e.g. an arm or leg phantom. The phantom may, for example, comprise a rigid cylinder. Typically, the cuff 14 can be supplied with a fluid (e.g. a gas, such as air, or any other fluid) suitable for inflating the cuff 14.

The cuff 14 referred to herein is configured to be worn on or around (e.g. wrapped around, attached to, or fastened to) the measurement site 20 of the subject 18 and/or a phantom of a body part. The measurement site 20 of the subject 18 can be any site on the body of the subject 18 that is suitable for use in measuring a blood pressure of the subject 18, such as any site on the body of the subject 18 that comprises an artery. The measurement site 20 of the subject 18 may be located on a limb of the subject, such as an arm (e.g. an upper arm or a forearm) of the subject 18. Thus, the cuff 14 can be configured to be worn on or around (e.g. wrapped around, attached to, or fastened to) a limb of the subject 18. In the embodiment illustrated in Fig. 4, the measurement site 20 of the subject 18 is located on the arm or, more specifically, the upper arm of the subject 18. Thus, the cuff 14 is worn on or around (e.g. wrapped around, attached to, or fastened to) the upper arm of the subject 18 in the embodiment illustrated in Fig. 4.

Although not illustrated in Fig. 4, alternatively or in addition to the cuff 14, the system 10 can comprise other components, such as the valve. In some embodiments, the cuff 14 may comprise the valve. In other embodiments, a tube (or hose) connected to the cuff 14 may comprise the valve.

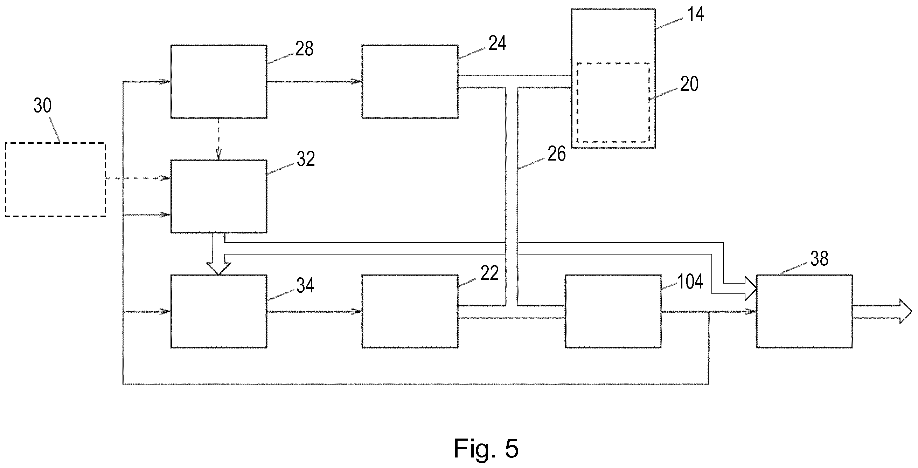

Fig. 5 illustrates a system according to an embodiment. The system comprises the cuff 14 described earlier that is inflatable to pressurize the measurement site 20 of the subject. The system also comprises the pump 24 described earlier, the tube (or hose) 26 described earlier, the valve 22 described earlier, and the at least one pressure sensor 104 described earlier. The cuff 14, the valve 22, the pump 24, and the at least one pressure sensor 104 are connected to the tube 26.

As mentioned earlier, the pump 24 is controllable to inflate the cuff 14. As also mentioned earlier, the at least one pressure sensor 104 is configured to measure pressure and pressure oscillations. More specifically, the at least one pressure sensor 104 can be configured to measure the pressure in the cuff 14 and the pressure oscillations in the cuff 14. Thus, the at least one pressure sensor 104 can be configured to obtain the pressure signal, the first oscillation signal and the second oscillation signal.

As illustrated in Fig. 5, in some embodiments, the system may also comprise the at least one other sensor 30 described earlier. As mentioned earlier, the at least one other sensor 30 can be configured to obtain data indicative of a cause of the at least one artifact during the period in which the cuff 14 is inflated.

In illustrated embodiment of Fig. 5, the one or more processors 102 of the apparatus 12 comprise the inflation controller 28 described earlier, the artifact detection module 32 described earlier, the deflation controller 34 described earlier, and the blood pressure measurement module 38 described earlier. Thus, in some embodiments, the system can comprise both an inflation-control system and a deflation-control system.

In the embodiment illustrated in Fig. 5, the inflation controller 28 controls the inflation of the cuff 14. In particular, the inflation controller 28 controls the pump 24 to control the inflation of the cuff 14. The at least one pressure sensor 104 obtains the pressure signal and the first oscillation signal. The artifact detection module 32 receives the pressure signal and the first oscillation signal from the at least one pressure sensor 104. The artifact detection module 32 detects whether the first oscillation signal comprises at least one artifact and, if the first oscillation signal is detected to comprise at least one artifact, identifies in the pressure signal, one or more pressures in the cuff 14 at which the at least one artifact is detected. In embodiments where the artifact detection module 32 is configured to acquire data from the at least one other sensor 30, the artifact detection module 32 may be configured to detect whether the first oscillation signal comprises at least one artifact based on the acquired data.

In the embodiment illustrated in Fig. 5, the deflation controller 34 controls the valve 22 to release pressure from the cuff 14 during the period in which the cuff 14 is deflated. The deflation controller 34 receives the artifact information (i.e. the one or more identified pressures at which the at least one artifact is detected) from the artifact detection module 32 to control the valve 22 during the period in which the cuff 14 is deflated, e.g. such that a constant or substantially constant pressure is maintained in the cuff 14 at the one or more pressures at which the at least one artifact is detected by the artifact detection module 32. The artifact detection module 32 may receive the second oscillation signal from the at least one pressure sensor 104. The blood pressure measurement module 38 receives the pressure signal, the first oscillation signal and the second oscillation signal from the at least one pressure sensor 104. The blood pressure measurement module 38 may also receive the detected artifact information (i.e. the one or more identified pressures at which the at least one artifact is detected) from the artifact detection module 32. The blood pressure measurement module 38 processes these signals (e.g. together with the detected artifact information) to measure the blood pressure of the subject.

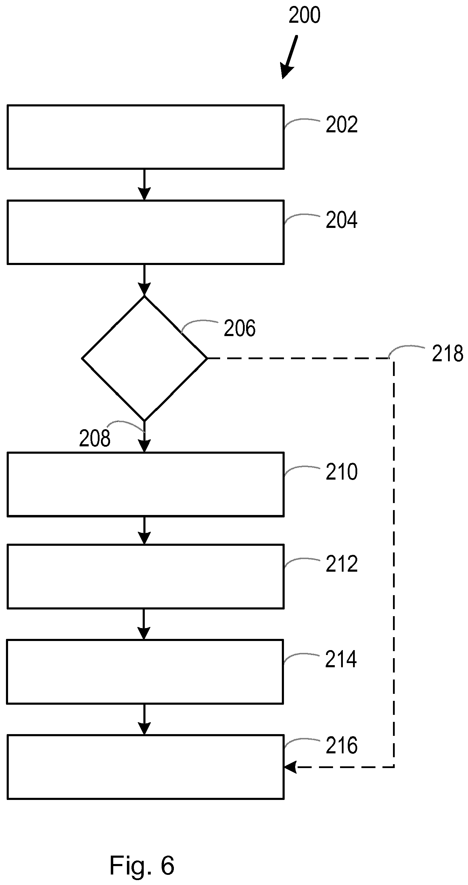

Fig. 6 illustrates a method 200 of operating the apparatus 12 described earlier according to an embodiment. The method 200 is a computer-implemented method. More specifically, the method 200 can generally be performed by or under the control of the one or more processors 102 of the apparatus 12 described earlier.

During the method 200 of Fig. 6, the cuff 14 is inflated to pressurize the measurement site 20 of the subject 18 and then subsequently deflated. The one or more processors 102 of the apparatus 12 (e.g. the inflation controller 28) can be configured to control the inflation of the cuff 14 and/or the one or more processors 102 of the apparatus 12 (e.g. the deflation controller 34) can be configured to control the deflation of the cuff 14. In some embodiments, the one or more processors 102 of the apparatus 12 (e.g. the inflation controller 28) can be configured to stop the inflation of the cuff 14 when pressure oscillations are no longer measurable (e.g. when the pressure oscillations have disappeared) or when the pressure in the cuff 14 reaches a predefined limit (e.g. a predefined safety limit). In these embodiments, the one or more processors 102 of the apparatus 12 (e.g. the deflation controller 34) can be configured to then start deflation of the cuff 14.

Although not illustrated in Fig. 6, in some embodiments, the one or more processors 102 of the apparatus 12 (e.g. the inflation controller 28) may be configured to control the inflation of the cuff 14 at a predetermined speed. In some embodiments, the one or more processors 102 of the apparatus 12 (e.g. the inflation controller 28) may be configured to control the speed of inflation of the cuff 14 based on a heart rate of the subject 18. In this way, it can be ensured that there are a sufficient amount of heart beats during the period in which the cuff 14 is inflated for blood pressure to be measured accurately.

Alternatively or in addition, in some embodiments, the one or more processors 102 of the apparatus 12 (e.g. the inflation controller 28) may be configured to control the inflation of the cuff 14 up to a maximum pressure prior to deflation. For example, the one or more processors 102 of the apparatus 12 (e.g. the inflation controller 28) may be configured to ramp the pressure up to the maximum pressure and then the one or more processors 102 of the apparatus 12 (e.g. the deflation controller 34) can be control the valve to deflate the cuff 14 in the manner described herein.

At block 202 of Fig. 6, a pressure signal is received. More specifically, the one or more processors 102 of the apparatus 12 receive the pressure signal. For example, the artifact detection module 32 may receive the pressure signal. The pressure signal may, for example, be received from the at least one pressure sensor 104. The received pressure signal is indicative of a pressure in the cuff 14 during a period in which the cuff 14 is inflated and subsequently deflated. In some embodiments, the received pressure signal can be a direct current (DC) component of the pressure signal. That is, the received pressure signal can be indicative of a DC pressure in the cuff 14 during the period in which the cuff 14 is inflated and subsequently deflated.

At block 204 of Fig. 6, a first oscillation signal is received. More specifically, the one or more processors 102 of the apparatus 12 receive the first oscillation signal. For example, the artifact detection module 32 may receive the first oscillation signal. The first oscillation signal may, for example, be received from the at least one pressure sensor 104. The first oscillation signal is indicative of pressure oscillations in the cuff 14 at (a plurality of) different pressures in the cuff 14 during the period in which the cuff 14 is inflated.

At block 206 of Fig. 6, it is detected whether the first oscillation signal comprises at least one artifact. More specifically, the one or more processors 102 of the apparatus 12 detect whether the first oscillation signal comprises at least one artifact. For example, the artifact detection module 32 may detect whether the first oscillation signal comprises at least one artifact. In some embodiments, the one or more processors 102 of the apparatus 12 (e.g. the artifact detection module 32) may be configured to acquire, from at least one other sensor 30, data indicative of a cause of the at least one artifact during the period in which the cuff 14 is inflated. In these embodiments, the one or more processors 102 of the apparatus 12 (e.g. the artifact detection module 32) may be configured to detect whether the first oscillation signal comprises at least one artifact based on the acquired data.

For example, in some embodiments, the one or more processors 102 of the apparatus 12 (e.g. the artifact detection module 32) can be configured to detect whether the first oscillation signal comprises at least one artifact based on the acquired data by being configured to compare the acquired data to any one or more of the pressure signal and the first oscillation signal, in order to detect whether the first oscillation signal comprises at least one artifact. As mentioned earlier, in some embodiments, the at least one other sensor 30 can comprise a motion sensor (e.g. an accelerometer) and/or an electrocardiogram (ECG) sensor. In embodiments involving a motion sensor, the acquired data can comprise motion data indicative of a motion of the cuff 14 during the period in which the cuff 14 is inflated. In embodiments involving an ECG sensor, the acquired data can comprise ECG data indicative of an irregular heart beat of the subject 18 during the period in which the cuff 14 is inflated.

As illustrated by arrow 208 of Fig. 6, if the first oscillation signal is detected to comprise at least one artifact, the method moves to block 210 of Fig. 6. At block 210 of Fig. 6, one or more pressures in the cuff 14 at which the at least one artifact is detected are identified in the pressure signal. More specifically, the one or more processors 102 of the apparatus 12 (e.g. the artifact detection module 32) identify, in the pressure signal, one or more pressures in the cuff 14 at which the at least one artifact is detected.

At block 212 of Fig. 6, a valve 22 is controlled to release pressure from the cuff 14 during a period in which the cuff 14 is deflated. More specifically, the one or more processors 102 of the apparatus 12 control the valve 22 in this way. For example, the deflation controller 34 may control the valve 22 in this way.

In some embodiments, the one or more processors 102 of the apparatus 12 (e.g. the deflation controller 34) may be configured to control the valve 22 to maintain a constant or substantially constant pressure in the cuff 14 during the period in which the cuff 14 is deflated when the pressure in the cuff 14 reaches the one or more identified pressures. Thus, the one or more processors 102 of the apparatus 12 (e.g. the deflation controller 34) may be configured to control the valve 22 to introduce (or create) a plateau or step in the pressure signal at the one or more identified pressures during the period in which the cuff 14 is deflated. One or more (first) processors 102 of the apparatus 12 (e.g. the artifact detection module 32) may obtain one or more desired plateaus or steps during the period in which the cuff 14 is inflated. One or more second processors 102 of the apparatus 12 (e.g. the deflation controller 34) may receive, from one or more first processors 102 (e.g. the artifact detection module 32), an indication of the plateau or step to introduce (or create) in the pressure signal at the one or more identified pressures during the period in which the cuff 14 is deflated to control the valve 22 accordingly. In this way, the pressure signal can comprise a single or a plurality (e.g. a few) plateaus or steps in the deflation phase.

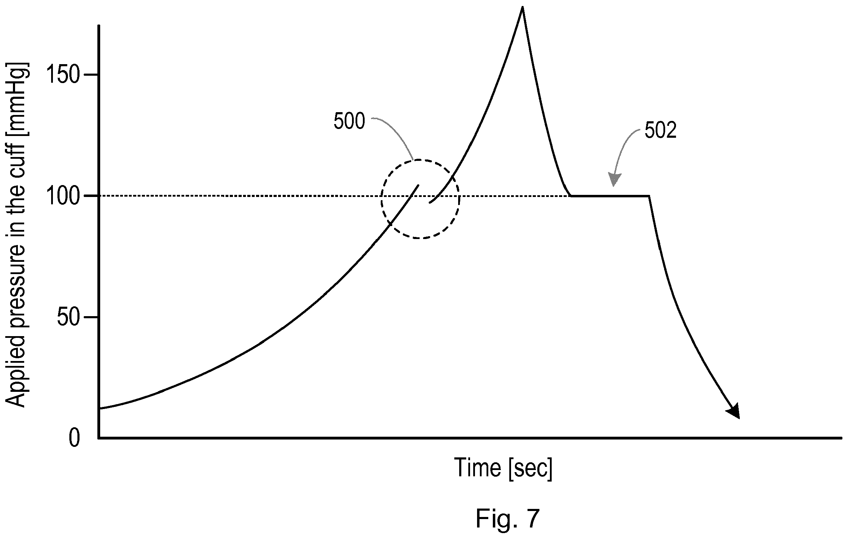

Fig. 7 is a graphical illustration of an example pressure signal received during a period in which the cuff 14 is deflated this manner. The pressure signal is indicative of the pressure in the cuff 14 during the period in which the cuff 14 is inflated and subsequently deflated. In Fig. 7, the (applied) pressure in the cuff 14 is shown on the vertical axis (in millimeter of mercury, mmHg) and the time is shown on the horizontal axis (in seconds, sec). For illustration purposes, the pressure signal is shown to comprise an artifact 500 that results in an invalid measurement of the pressure oscillation in the first oscillation signal during a time period in which this artifact occurs. Although only one artifact 500 is shown, it will be understood that the first oscillation signal can comprise more than one artifact.

As illustrated in Fig. 7, in some embodiments, the one or more processors 102 of the apparatus 12 (e.g. the inflation controller 28) may be configured to (e.g. control the pump 24 to) inflate the cuff 14 continuously during the period in which the cuff 14 is inflated. In these embodiments, it can be said that the inflation ramp is continuous. That is, the inflation ramp does not have plateaus or steps. In this way, the inflation can be performed as quickly as possible to avoid prolonged discomfort for the subject 18.

As also illustrated in Fig. 7, in some embodiments, the one or more processors 102 of the apparatus 12 (e.g. the deflation controller 34) may be configured to control the valve 22 to release pressure from the cuff 14 continuously during the period in which the cuff 14 is deflated apart from one or more time periods 502 in which the constant or substantially constant pressure is maintained in the cuff 14. In this way, the cuff 14 is deflated as quickly as possible with a plateau or step only at the one or more identified pressures at which at least one artifact is detected during inflation. The number of plateaus or steps can be limited.

In the example illustrated in Fig. 7, a single artifact 500 is identified at a pressure of roughly 100mmHg during the period in which the cuff 14 is inflated and thus a plateau or step is introduced (or created) in the pressure signal at the same pressure of 100mmHg during the period in which the cuff 14 is deflated. Aside from this plateau or step, the cuff 14 can otherwise be deflated quickly.

In effect, in some embodiments, the one or more processors 102 of the apparatus 12 (e.g. the deflation controller 34) may be configured to control the valve 22 to pause deflation, such that more pressure oscillations can be obtained.

In some embodiments, the one or more processors 102 of the apparatus 12 (e.g. the deflation controller 34) may be configured to control the valve 22 to maintain the constant or substantially constant pressure in the cuff 14 for a time period that covers at least one pressure oscillation that is free from artifacts (e.g. at least two pressure oscillations that are free from artifacts) and/or that covers at least one heart cycle (e.g. at least two heart cycles). For example, the one or more processors 102 of the apparatus 12 (e.g. the deflation controller 34) may be configured to control the valve 22 to introduce (or create) each plateau or step in the pressure signal for a time period that covers at least one pressure oscillation that is free from artifacts (e.g. at least two pressure oscillations that are free from artifacts) and/or that covers at least one heart cycle (e.g. at least two heart cycles). In this way, the each plateau or step will have a sufficient length.

An advantage of having multiple heart cycles in each plateau or step is that the consistency of pressure oscillations can be assessed. Thus, in some embodiments, the one or more processors 102 of the apparatus 12 can be configured to detect whether pressure oscillations are consistent. In some embodiments, if pressure oscillations are inconsistent, the length of the plateau or step can be extended. In some of these embodiments, the one or more processors 102 of the apparatus 12 (e.g. the deflation controller 34) may be configured to control the valve 22 to release pressure from the cuff 14 continuously during the period in which the cuff 14 is deflated apart from one or more time periods in which a plateau or step is introduced (or created). In other embodiments, the pressure signal may comprise a plurality of plateaus or steps, where each plateau or step introduced (or created) at the one or more identified pressures may be longer than (or extended compared to) the other plateaus or steps.

Returning back to Fig. 6, at block 214, a second oscillation signal is received. More specifically, the one or more processors 102 of the apparatus 12 receive the second oscillation signal. For example, the blood pressure measurement module 38 may receive the second oscillation signal. The second oscillation signal may, for example, be received from the at least one pressure sensor 104. The second oscillation signal is indicative of pressure oscillations in the cuff 14 at the one or more identified pressures during the period in which the cuff 14 is deflated.

Thus, in the manner described, pressure oscillations are measured during inflation and then more pressure oscillations can be measured during deflation. In effect, for pressure oscillations where artifacts are detected during inflation, the pressure oscillations are re-measured during deflation.

At block 216 of Fig. 6, the pressure signal, the first oscillation signal and the second oscillation signal are processed to measure the blood pressure of a subject 18. More specifically, the one or more processors 102 of the apparatus 12 process the pressure signal, the first oscillation signal and the second oscillation signal to measure the blood pressure of the subject 18. For example, the blood pressure measurement module 38 may process the pressure signal, the first oscillation signal and the second oscillation signal to measure the blood pressure of the subject 18. In some embodiments, the blood pressure of a subject 18 may be measured when the full cycle of inflation, artifact detection and deflation is complete, e.g. when blocks 202 to 214 of Fig. 6 are complete. In this way, a complete set of pressure oscillations is obtained for use in the measurement of blood pressure of the subject 18 once deflation is complete (and, for example, after a merger of pressure oscillations from the time period in which the cuff 14 is inflated and pressure oscillations from the time period in which the cuff 14 is subsequently deflated).

Herein, the measured blood pressure of the subject 18 can comprise a mean arterial pressure (MAP) of the subject 18, a systolic blood pressure (SBP) of the subject 18 and/or a diastolic blood pressure (DBP) of the subject 18. The measured blood pressure of the subject 18 referred to herein may also be referred to as a measured blood pressure value for the subject 18.

The one or more processors 102 of the apparatus 12 (e.g. the blood pressure measurement module 38) may be configured to process the pressure signal and the first and second oscillation signals to measure the blood pressure of the subject 18 in any suitable way. In some embodiments, the one or more processors 102 of the apparatus 12 (e.g. the blood pressure measurement module 38) may be configured to process the pressure signal and the first and second oscillation signals to measure pressure oscillation amplitudes at the different pressures in the cuff 14 and measure the blood pressure of the subject 18 based on the measured pressure oscillation amplitudes.

For example, in some embodiments, the one or more processors 102 of the apparatus 12 (e.g. the blood pressure measurement module 38) may be configured to measure, from the first oscillation signal, a plurality of first pressure oscillation amplitudes at the different pressures in the cuff 14 and measure, from the second oscillation signal, one or more second pressure oscillation amplitudes at the one or more identified pressures in the cuff 14. In these embodiments, the one or more processors 102 of the apparatus 12 (e.g. the blood pressure measurement module 38) can be configured to combine (or merge) the one or more second pressure oscillation amplitudes with the plurality of first pressure oscillation amplitudes to obtain a set of combined (or merged) pressure oscillation amplitudes at the different pressures in the cuff 14 and process the set of combined (or merged) pressure oscillation amplitudes at the different pressures in the cuff 14 to measure the blood pressure of the subject 18.

In effect, a plurality of pressure oscillation amplitudes are obtained, which correspond to a plurality of pressures in the cuff 14. More specifically, each of the plurality of pressure oscillation amplitudes correspond to (a different) one of the plurality of pressures in the cuff 14. The plurality of pressure oscillation amplitudes comprise the first pressure oscillation amplitudes complimented with the second pressure oscillation amplitudes. In this way, each of the plurality of pressures in the cuff 14 has a corresponding pressure oscillation amplitude. It is thus possible to obtain pressure oscillation amplitudes for the complete plurality of pressures in the cuff 14.

In some of these embodiments, the one or more processors 102 of the apparatus 12 (e.g. the blood pressure measurement module 38) may be configured to combine the one or more second pressure oscillation amplitudes with the plurality of first pressure oscillation amplitudes by being configured to replace one or more of the plurality of first pressure oscillation amplitudes at the one or more identified pressures in the cuff 14 with the one or more corresponding second pressure oscillation amplitudes to obtain the set of combined pressure oscillation amplitudes.

In some embodiments, the one or more processors 102 of the apparatus 12 (e.g. the blood pressure measurement module 38) can be configured to replace the one or more of the plurality of first pressure oscillation amplitudes by being configured to compare the one or more of the plurality of first pressure oscillation amplitudes to the one or more corresponding second pressure oscillation amplitudes and, for each discrepancy identified from the comparison, replace the one or more of the plurality of first pressure oscillation amplitudes with the one or more corresponding second pressure oscillation amplitudes. In these embodiments, a discrepancy may, for example, be identified when there is a significant difference (e.g. a difference that exceeds a predefined threshold) between the first pressure oscillation amplitude and the corresponding second pressure oscillation amplitude. The difference can, for example, be in the amplitude of and/or timing of the pressure oscillations. In this way, it is possible to detect any outliers in the first pressure oscillation amplitudes with respect to corresponding second pressure oscillation amplitudes.

In other embodiments, the one or more processors 102 of the apparatus 12 (e.g. the blood pressure measurement module 38) can be configured to replace the one or more of the plurality of first pressure oscillation amplitudes by being configured to compare the one or more of the plurality of first pressure oscillation amplitudes with their neighboring first pressure oscillation amplitudes and, for each discrepancy identified from the comparison, replace the one or more of the plurality of first pressure oscillation amplitudes with the one or more corresponding second pressure oscillation amplitudes. In these embodiments, a discrepancy may, for example, be identified between a first pressure oscillation amplitude and a neighboring first pressure oscillation amplitude when there is a significant difference (e.g. a difference that exceeds a predefined threshold) between the first pressure oscillation amplitude and the neighboring first pressure oscillation amplitude. The difference can, for example, be in the amplitude and/or timing of (e.g. time between) the pressure oscillations. In this way, it is possible to detect any outliers in the first pressure oscillation amplitudes with respect to neighboring first pressure oscillation amplitudes. In any of the embodiments, a first pressure oscillation amplitude in respect of which a discrepancy is identified can be marked or flagged as unreliable.

As described earlier, in some embodiments, a constant or substantially constant pressure may be maintained in the cuff 14 (e.g. a plateau or step may be introduced in the pressure signal) for a time period that covers at least two second pressure oscillations that are free from artifacts in the second oscillation signal. In some of these embodiments, the one or more processors 102 of the apparatus 12 (e.g. the blood pressure measurement module 38) may be configured to, if the at least two second pressure oscillation amplitudes are close to each other in amplitude (e.g. differ from each other by less than a predefined amount) but not close to the corresponding first oscillation amplitude (e.g. differ from the corresponding first oscillation amplitude by more than the predefined amount), determine an average of the at least two second pressure oscillations. In these embodiments, the one or more processors 102 of the apparatus 12 (e.g. the blood pressure measurement module 38) can be configured to replace the first pressure oscillation amplitude with the determined average. In other embodiments, the one or more processors 102 of the apparatus 12 (e.g. the blood pressure measurement module 38) may be configured to, if the at least two second pressure oscillation amplitudes are as close to each other in amplitude as they are to the corresponding first oscillation amplitude (e.g. differ from each other and the corresponding first oscillation amplitude by the same amount or approximately the same amount), determine a mean or median of the at least two second pressure oscillation amplitudes and the corresponding first oscillation amplitude. In these embodiments, the one or more processors 102 of the apparatus 12 (e.g. the blood pressure measurement module 38) can be configured to replace the first pressure oscillation amplitude with the determined mean or median.

Thus, the blood pressure of the subject 18 can be measured from the set of combined pressure oscillation amplitudes at the different pressures in the cuff 14 according to some embodiments. The one or more processors 102 of the apparatus 12 (e.g. the blood pressure measurement module 38) may be configured to measure the blood pressure of the subject 18 from the set of combined pressure oscillation amplitudes in any suitable way. For example, the MAP can be measured by identifying the pressure in the cuff 14 at which a maximum oscillation amplitude occurs in the combined pressure oscillation amplitudes. Alternatively, a pressure oscillation envelope may be determined for the combined pressure oscillation amplitudes. The pressure oscillation envelope outlines the extremes of the pressure oscillation amplitudes. The pressure oscillation envelope can be determined by plotting the discretely measured pressure oscillation amplitudes as function of the pressure in the cuff 14 and fitting a curve through these discretely measured pressure oscillation amplitudes. Thus, the pressure oscillation envelope can be determined by fitting a curve to the extremes of the pressure oscillation amplitudes. In embodiments involving a pressure oscillation envelope, the MAP can be measured by identifying the pressure in the cuff 14 at which a maximum occurs in the pressure oscillation envelope.

The SBP of the subject 18 can be measured by identifying the cuff pressure that is larger than the MAP and where the pressure oscillation amplitude in the combined pressure oscillation amplitudes has a fixed ratio (e.g. 0.4) or a fixed percentage (e.g. 40%) of the maximum pressure oscillation amplitude in the combined pressure oscillation amplitudes. The DBP of the subject 18 can be measured by identifying the cuff pressure that is smaller than the MAP and where the pressure oscillation amplitude in the combined pressure oscillation amplitudes has a fixed ratio (e.g. 0.8) or fixed percentage (e.g. 80%) of the maximum oscillation amplitude in the combined pressure oscillation amplitudes. The fixed ratios used in the measurement of the SBP and DBP are selected in such a way that the measured value of the MAP satisfies the formula: MAP = 2/3 ^{∗} DBP + 1/3 ^{∗} SBP.

Thus, in this way, after deflation of the cuff 14 (and, for example, after the combining of the pressure oscillations from inflation and deflation phase), a complete or at least more complete set of pressure oscillations can be processed to measure the blood pressure of the subject 18. Nevertheless, in some embodiments, the one or more processors 102 of the apparatus 12 (e.g. the blood pressure measurement module 38) may be configured to determine the total number of pressure oscillations and, if the total number of pressure oscillations is determined to be below a predefined threshold, abort measuring the blood pressure of the subject 18. In some of these embodiments, the one or more processors 102 of the apparatus 12 (e.g. the blood pressure measurement module 38) may be configured to output (e.g. via the communications interface 110 of the apparatus 12) an indication that the blood pressure measurement is aborted. In other embodiments, the one or more processors 102 of the apparatus 12 (e.g. the blood pressure measurement module 38) may be configured to measure the blood pressure of the subject 18 even if the total number of pressure oscillations is determined to be below the predefined threshold. However, in these embodiments, the one or more processors 102 of the apparatus 12 (e.g. the blood pressure measurement module 38) may be configured to output (e.g. via the communications interface 110 of the apparatus 12) an indication that the blood pressure measurement is invalid.

Returning back to Fig. 6, as illustrated by arrow 218, in some embodiments, if the first oscillation signal is detected to be free from artifacts, the method may move to block 216 of Fig. 6 (instead of moving to block 210 of Fig. 6). In this case, at block 216 of Fig. 6, the pressure signal and the first oscillation signal are processed to measure the blood pressure of a subject 18. More specifically, the one or more processors 102 of the apparatus 12 process the pressure signal and the first oscillation signal to measure the blood pressure of the subject 18. For example, the blood pressure measurement module 38 may process these signals to measure the blood pressure of a subject 18. In some embodiments, the one or more processors 102 of the apparatus 12 (e.g. the blood pressure measurement module 38) may be configured to measure, from the first oscillation signal, a plurality of first pressure oscillation amplitudes at the different pressures in the cuff 14 and process the first pressure oscillation amplitudes at the different pressures in the cuff 14 to measure the blood pressure of the subject 18, such as the SBP of the subject 18, the DBP of the subject 18 and/or the MAP of the subject 18 in the manner described earlier.

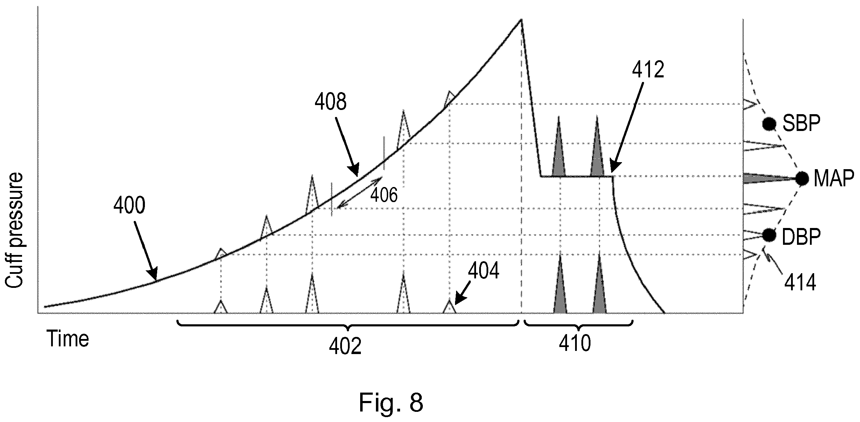

Fig. 8 is a graphical illustration of an example pressure signal 400 received during a period in which the cuff 14 is inflated and subsequently deflated in the manner described herein. The pressure signal is indicative of the pressure in the cuff 14 during the period in which the cuff 14 is inflated and subsequently deflated. The pressure signal 400 is plotted together with a first oscillation signal 402 during the period in which the cuff 14 is inflated. The first oscillation signal 402 is indicative of pressure oscillations in the cuff 14 at different pressures in the cuff 14 during the period in which the cuff 14 is inflated. The pressure signal 400 is plotted together with a plurality of first pressure oscillation amplitudes 404 at the different pressures in the cuff 14, which are measured from the first oscillation signal.

As illustrated in Fig. 8, the first oscillation signal 402 comprises an artifact 406. Thus, according to the method described herein, the first oscillation signal 402 is detected to comprise at least one artifact. It can be seen from the plurality of first pressure oscillation amplitudes 404 in Fig. 8 that the oscillation amplitudes at the pressures in the cuff 14 at which the artifact occurs in the first oscillation signal 402 are missing and thus the first pressure oscillation amplitudes 404 are inaccurate at the moments of the artifact 406. As such, according to the method described herein, the pressure 408 in the cuff 14 at which the artifact 406 is detected is identified in the pressure signal 400, the valve 22 is controlled to release pressure from the cuff 14 during the period in which the cuff 14 is deflated and a second oscillation signal 410 is received. The second oscillation signal 410 is indicative of pressure oscillations in the cuff 14 at the identified pressure during the period in which the cuff 14 is deflated.

As illustrated in Fig. 8, the valve 22 is controlled to maintain a constant or substantially constant pressure in the cuff 14 during the period in which the cuff 14 is deflated when the pressure in the cuff 14 reaches the identified pressure 408. More specifically, in this example, the valve 22 is controlled to introduce a plateau or step 412 in the pressure signal 400 at the identified pressure 408 during the period in which the cuff 14 is deflated. The plateau or step 412 introduced at the identified pressure 408 is long enough to cover at least one pressure oscillation that is free from artifacts (or, in this example, to cover two pressure oscillations that are free from artifacts). In effect, the valve 22 is controlled to pause deflation at the identified pressure 408 in order for more pressure oscillations to be obtained.

In the manner described earlier, the pressure signal 400, the first oscillation signal 402 and the second oscillation signal 410 can be processed to measure the blood pressure of the subject 18, such as any one or more of the SBP of the subject 18, MAP of the subject 18, and DBP of the subject 18. For example, in the manner described earlier, the blood pressure of the subject 18 may be measured from the set of combined (or merged) pressure oscillation amplitudes or from an envelope 414 determined for the set of combined (or merged) pressure oscillation amplitudes.

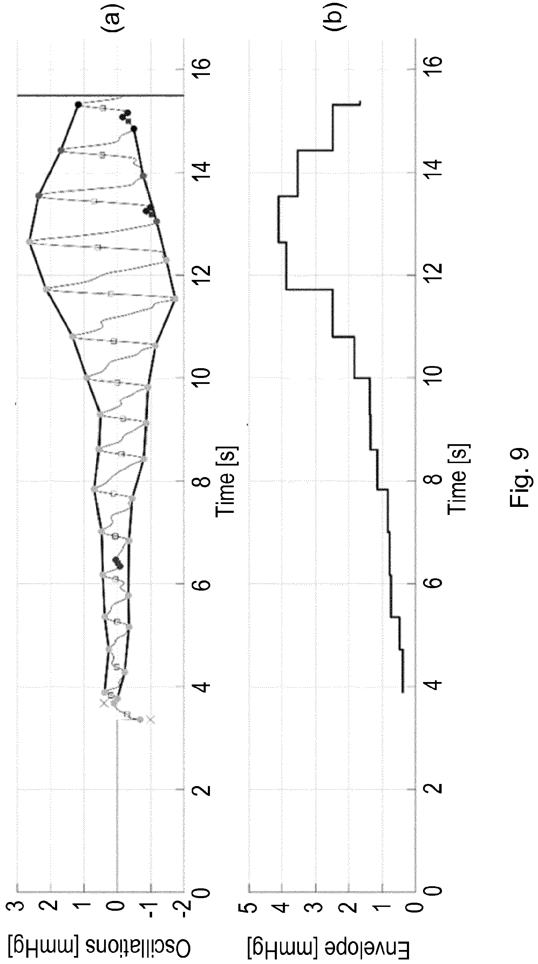

Figs. 9, 10 and 11 are illustrative of some of the issues with existing techniques, which can be overcome by way of the technique described herein.

Fig. 9 is a graphical illustration of a pressure oscillation signal and a corresponding pressure envelope from a real inflation-based blood pressure measurement. In more detail, Fig. 9(a) shows the pressure oscillations in the cuff 14 and the constructed pressure envelope as function of time. The constructed pressure envelope outlines the extremes of the pressure oscillation signal. In Fig. 9(a), the pressure oscillations in the cuff 14 are shown on the vertical axis (in millimeter of mercury, mmHg) and the time is shown on the horizontal axis (in seconds, s).

Fig. 9(b) shows the final pressure envelope as function of time. In Fig. 9(b), the final pressure envelope is shown on the vertical axis (in millimeter of mercury, mmHg) and the time is shown on the horizontal axis (in seconds, s). The final pressure envelope is indicative of the pressure oscillation amplitudes, which are measured from the pressure oscillation signal of Fig. 9(a). The final pressure envelope is used in measuring the blood pressure. As illustrated in Fig. 9(b), in the absence of artifacts, an accurate final pressure envelope is obtained and consequently reliable blood pressure measurements can be derived. In this example, the blood pressure (systolic/diastolic) measurement is 126/78 mmHg.

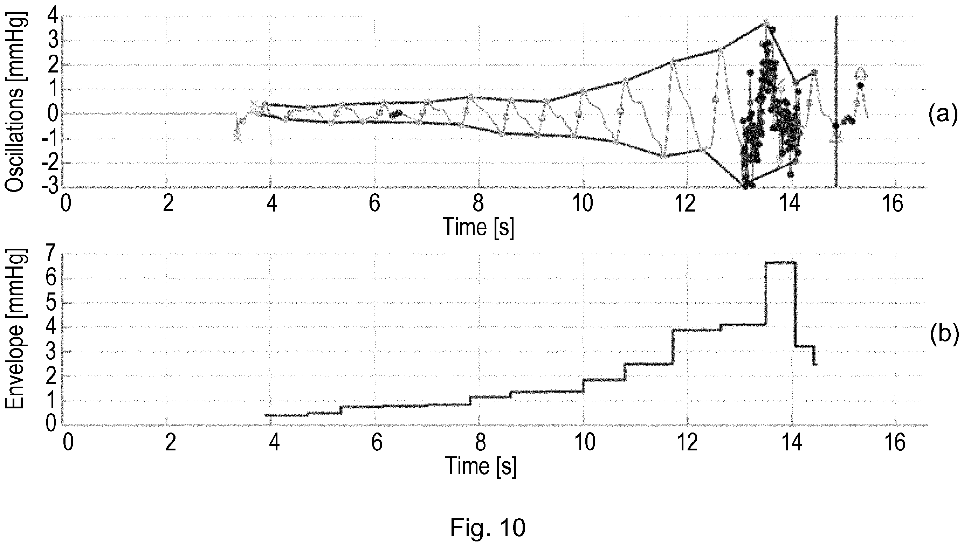

Fig. 10 is a graphical illustration of a pressure oscillation signal and a corresponding pressure envelope from an artificial inflation-based blood pressure measurement. The inflation-based blood pressure measurement is artificial in that artifacts are numerically introduced into the same inflation-based blood pressure measurement of Fig. 9 in order to arrive at Fig. 10. In more detail, Fig. 10(a) shows the pressure oscillations with the numerically introduced artifacts and the constructed pressure envelope as function of time. The constructed pressure envelope outlines the extremes of the pressure oscillation signal. In Fig. 10(a), the pressure oscillations in the cuff 14 are shown on the vertical axis (in millimeter of mercury, mmHg) and the time is shown on the horizontal axis (in seconds, s).

Fig. 10(b) shows the final pressure envelope as function of time. In Fig. 10(b), the final pressure envelope is shown on the vertical axis (in millimeter of mercury, mmHg) and the time is shown on the horizontal axis (in seconds, s). The final pressure envelope is indicative of the pressure oscillation amplitudes, which are measured from the pressure oscillation signal of Fig. 10(a). The final pressure envelope is used in measuring the blood pressure. As illustrated in Fig. 10(b), when the pressure oscillation signal comprises artifacts, the final pressure envelope is distorted. This leads to unreliable (e.g. incorrect) blood pressure measurements. Also, it is not always possible to simply reject the regions of artifacts to compute the pressure envelope and measure the blood pressure, since rejection of the artifacts region may lead to too few oscillations. In this example, the blood pressure (systolic/diastolic) measurement is 119/99 mmHg, which deviates from the original measurement of 126/78 mmHg.

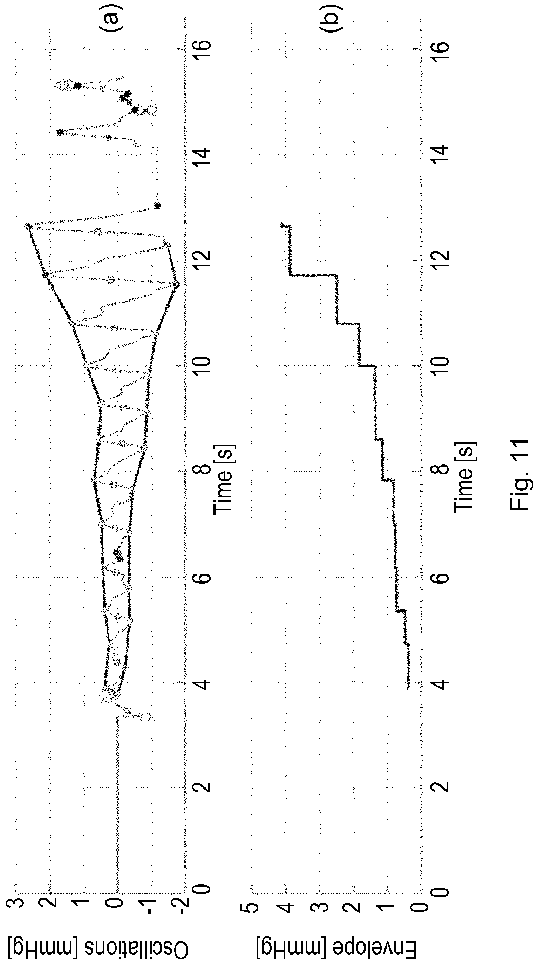

Fig. 11 is a graphical illustration of a pressure oscillation signal and a corresponding pressure envelope from an artificial inflation-based blood pressure measurement. The inflation-based blood pressure measurement is artificial in that artifacts are numerically introduced into the same inflation-based blood pressure measurement of Fig. 9 in order to arrive at Fig. 11. In more detail, Fig. 11(a) shows the pressure oscillations with the numerically introduced artifacts and the constructed pressure envelope as function of time as in Fig. 10. The constructed pressure envelope outlines the extremes of the pressure oscillation signal. In Fig. 11(a), the pressure oscillations in the cuff 14 are shown on the vertical axis (in millimeter of mercury, mmHg) and the time is shown on the horizontal axis (in seconds, s). However, in Fig. 11(a), the artifacts are blanked in the pressure oscillation signal for the blood pressure measurement.

Fig. 11(b) shows the final pressure envelope as function of time. In Fig. 11(b), the final pressure envelope is shown on the vertical axis (in millimeter of mercury, mmHg) and the time is shown on the horizontal axis (in seconds, s). The final pressure envelope is indicative of the pressure oscillation amplitudes, which are measured from the pressure oscillation signal of Fig. 11(a). The final pressure envelope is used in measuring the blood pressure. As illustrated in Fig. 11(b), in view of the blanking of the artifacts, there are not enough pressure oscillations available to construct the final envelope in order to derive reliable blood pressure measurements.

The apparatus 12 and method 200 described herein overcome the issues illustrated by way of Figs. 9, 10 and 11. In particular, according to the apparatus 12 and method 200 described herein, reliable pressure oscillations are obtained at each of the different pressures in the cuff 14, either during the period in which the cuff 14 is inflated or otherwise during the period in which the cuff 14 is deflated. The pressure oscillations of the second oscillation signal obtained during the period in which the cuff 14 is deflated can be used to augment the pressure oscillations of the first oscillation signal obtained during the period in which the cuff 14 is inflated. It can thus be ensured that any blood pressure measured using the first oscillation signal and the second oscillation signal is reliable, whilst the measurement time is limited.

In some embodiments, any one or more of the steps of the method described herein may be performed before or during the blood pressure measurement. Alternatively or in addition, in some embodiments, at least one or all of the steps of the method described herein can be automated.

There is also described a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein an improved technique for measuring blood pressure, which addresses the limitations associated with the existing techniques. The technique provides an improvement to the existing inflation-based oscillometric blood pressure measurement technique.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (12) for use with a wearable cuff (14) in measuring blood pressure, wherein the cuff (14) is inflatable to pressurize a measurement site (20) of a subject (18) and the apparatus (12) comprises:
one or more processors (102) configured to:
receive a pressure signal indicative of a pressure in the cuff (14) during a period in which the cuff (14) is inflated and subsequently deflated;
receive a first oscillation signal indicative of pressure oscillations in the cuff (14) at different pressures in the cuff (14) during the period in which the cuff (14) is inflated;
detect whether the first oscillation signal comprises at least one artifact;
if the first oscillation signal is detected to comprise at least one artifact:
identify, in the pressure signal, one or more pressures in the cuff (14) at which the at least one artifact is detected;
control a valve (22) to release pressure from the cuff (14) during the period in which the cuff (14) is subsequently deflated; and
receive a second oscillation signal indicative of pressure oscillations in the cuff (14) at the one or more identified pressures during the period in which the cuff (14) is subsequently deflated; and
process the pressure signal, the first oscillation signal and the second oscillation signal to measure a blood pressure of a subject (18).

2. The apparatus (12) as claimed in claim 1, wherein:
the one or more processors (102) are configured to:
control the valve (22) to maintain a constant or substantially constant pressure in the cuff (14) during the period in which the cuff (14) is subsequently deflated when the pressure in the cuff (14) reaches the one or more identified pressures.

3. The apparatus (12) as claimed in claim 2, wherein:
the one or more processors (102) are configured to:
control the valve (22) to release pressure from the cuff (14) continuously during the period in which the cuff (14) is subsequently deflated apart from one or more time periods in which the constant or substantially constant pressure is maintained in the cuff (14).

4. The apparatus (12) as claimed in claim 2 or 3, wherein:
the one or more processors (102) are configured to:
control the valve (22) to maintain the constant or substantially constant pressure in the cuff (14) for a time period that covers at least one pressure oscillation that is free from artifacts and/or that covers at least one heart cycle.

5. The apparatus (12) as claimed in any of the preceding claims, wherein:
the one or more processors (102) are configured to process the pressure signal and the first and second oscillation signals to measure the blood pressure of the subject (18) by being configured to:
measure, from the first oscillation signal, a plurality of first pressure oscillation amplitudes at the different pressures in the cuff (14);
measure, from the second oscillation signal, one or more second pressure oscillation amplitudes at the one or more identified pressures in the cuff (14);
combine the one or more second pressure oscillation amplitudes with the plurality of first pressure oscillation amplitudes to obtain a set of combined pressure oscillation amplitudes at the different pressures in the cuff (14); and
process the set of combined pressure oscillation amplitudes at the different pressures in the cuff (14) to measure the blood pressure of the subject (18).

6. The apparatus (12) as claimed in claim 5 wherein:
the one or more processors (102) are configured to combine the one or more second pressure oscillation amplitudes with the plurality of first pressure oscillation amplitudes by being configured to:
replace one or more of the plurality of first pressure oscillation amplitudes at the one or more identified pressures in the cuff (14) with the one or more corresponding second pressure oscillation amplitudes to obtain the set of combined pressure oscillation amplitudes.

7. The apparatus (12) as claimed in claim 6, wherein:
the one or more processors (102) are configured to replace the one or more of the plurality of first pressure oscillation amplitudes by being configured to:
compare the one or more of the plurality of first pressure oscillation amplitudes to the one or more corresponding second pressure oscillation amplitudes; and
for each discrepancy identified from the comparison, replace the one or more of the plurality of first pressure oscillation amplitudes with the one or more corresponding second pressure oscillation amplitudes.

8. The apparatus (12) as claimed in any of the preceding claims, wherein:
the one or more processors (102) are configured to:
acquire, from at least one sensor (30), data indicative of a cause of the at least one artifact during the period in which the cuff (14) is inflated; and
detect whether the first oscillation signal comprises at least one artifact based on the acquired data.

9. The apparatus (12) as claimed in claim 8, wherein:
the one or more processors (102) are configured to detect whether the first oscillation signal comprises at least one artifact based on the acquired data by being configured to:
compare the acquired data to the pressure signal and/or the first oscillation signal to detect whether the first oscillation signal comprises at least one artifact.

10. The apparatus (12) as claimed in claim 8 or 9, wherein:
the at least one sensor (30) comprises a motion sensor and the acquired data comprises motion data indicative of a motion of the cuff (14) during the period in which the cuff (14) is inflated; and/or
the at least one sensor (30) comprises an electrocardiogram sensor and the acquired data comprises electrocardiogram data indicative of an irregular heart beat of the subject (18) during the period in which the cuff (14) is inflated.

11. The apparatus (12) as claimed in any of the preceding claims, wherein:
the one or more processors (102) are configured to:
control the inflation of the cuff (14) at a predetermined speed and/or up to a maximum pressure prior to subsequent deflation.

12. The apparatus (12) as claimed in any of the preceding claims, wherein:
the measured blood pressure of the subject (18) comprises a mean arterial pressure of the subject (18), a systolic blood pressure of the subject (18) and/or a diastolic blood pressure of the subject (18).

13. A system (10) comprising:
an apparatus (12) as claimed in any of the preceding claims; and
any one or more of the cuff (14) and the valve (22).

14. A method (200) of operating an apparatus for use with a wearable cuff in measuring blood pressure, wherein the cuff is inflatable to pressurize a measurement site of a subject and the method (200) comprises:
receiving (202) a pressure signal indicative of a pressure in the cuff during a period in which the cuff is inflated and subsequently deflated;
receiving (204) a first oscillation signal indicative of pressure oscillations in the cuff at different pressures in the cuff during the period in which the cuff is inflated;
detecting (206) whether the first oscillation signal comprises at least one artifact;
if (208) the first oscillation signal is detected to comprise at least one artifact:
identifying (210), in the pressure signal, one or more pressures in the cuff at which the at least one artifact is detected;
controlling (212) a valve to release pressure from the cuff during the period in which the cuff is subsequently deflated; and
receiving (214) a second oscillation signal indicative of pressure oscillations in the cuff at the one or more identified pressures during the period in which the cuff is subsequently deflated;
processing (216) the pressure signal, the first oscillation signal and the second oscillation signal to measure a blood pressure of the subject.

15. A computer program product comprising a computer readable medium, the computer readable medium having a computer readable code embodied therein, the computer readable code being configured such that, on execution by a suitable computer or processor, the computer or processor is caused to operate the apparatus according to the method as claimed in claim 14.
